(19) Europäisches Patentamt

European Patent Office

Office européen des brevets

(11) **EP 2 119 775 B1**

(12) **FASCICULE DE BREVET EUROPEEN**

(45) Date de publication et mention
de la délivrance du brevet:
**20.02.2013 Bulletin 2013/08**

(51) Int Cl.:
***C12N 11/04*** *(2006.01)*

(21) Numéro de dépôt: **09290349.1**

(22) Date de dépôt: **12.05.2009**

(54) **Dispositif, sytème et procédé microfluidique pour l'encapsulation contrôlée de particules ou amas de particules**

Vorrichtung, System und Mikrofluidverfahren zur kontrollierten Einkapselung von Partikeln oder Partikelklumpen

Microfluid device, system and method for controlled encapsulation of particles or clusters of particles

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **13.05.2008 FR 0802578**

(43) Date de publication de la demande:
**18.11.2009 Bulletin 2009/47**

(73) Titulaire: **Commissariat à l'Énergie Atomique et aux Énergies Alternatives
75015 Paris (FR)**

(72) Inventeurs:
• **Rivera, Florence
38240 Meylan (FR)**
• **Berthier, Jean
38240 Meylan (FR)**
• **Le Vot, Sophie
38800 Le Pont de Claix (FR)**

(74) Mandataire: **Priori, Enrico et al
Cabinet Orès
36, rue de St. Pétersbourg
75008 Paris (FR)**

(56) Documents cités:
**WO-A-2005/103106        US-A1- 2006 051 329
US-A1- 2007 009 668**

• **SUGIURA S ET AL: "Size control of calcium alginate beads containing living cells using micro-nozzle array" 1 juin 2005 (2005-06-01), BIOMATERIALS, ELSEVIER SCIENCE PUBLISHERS BV., BARKING, GB, PAGE(S) 3327 - 3331 , XP004684998 ISSN: 0142-9612 * page 3328, colonne de gauche, alinéa 4 - page 3330, colonne de droite, alinéa 1 ***

**Description**

L'invention porte sur un dispositif, un système et un procédé microfluidique pour l'encapsulation contrôlée de particules de dimensions sub-millimétriques ou d'amas de telles particules. L'invention convient plus particulièrement à l'encapsulation de cellules, notamment humaines, ou d'amas de cellules, tels que des îlots de Langerhans.

L'encapsulation cellulaire est une technique qui consiste à immobiliser des cellules ou des amas de cellules dans des microcapsules, de façon à les protéger des attaques du système immunitaire lors d'une transplantation. La porosité des capsules doit permettre l'entrée des molécules de faible poids moléculaire essentielles au métabolisme des cellules encapsulées, telles que des molécules de nutriments, d'oxygène, etc., tout en empêchant l'entrée de substances de poids moléculaire plus élevé comme les anticorps ou les cellules du système immunitaire. Cette perméabilité sélective des capsules est ainsi conçue pour assurer l'absence de contact direct entre les cellules encapsulées du donneur et celles du système immunitaire du receveur de la transplantation, ce qui permet de limiter les doses de traitement immunosuppresseur utilisées lors de la transplantation, ledit traitement immunosuppresseur présentant des effets secondaires lourds. Outre leur perméabilité sélective, les capsules produites doivent être biocompatibles, résistantes mécaniquement, et de taille adaptée a l'objet à encapsuler.

Parmi les multiples applications de l'encapsulation, on peut citer celle des îlots de Langerhans, amas de cellules fragiles situés dans le pancréas et constitués de plusieurs types cellulaires dont les cellules β qui régulent la glycémie dans le corps par production de l'insuline. L'encapsulation de ces îlots est une alternative aux thérapies cellulaires classiques (par exemple, transplantation de pancréas ou d'îlots) utilisées pour soigner le diabète insulinodépendant, maladie auto-immune dans laquelle le système immunitaire détruit ses propres cellules β productrices d'insuline.

Les principales méthodes d'encapsulation connues utilisent au choix:

- un jet d'air ou de liquide coaxial, les capsules produites ayant une taille variant entre 400 μm et 800 μm (cependant la taille moyenne des capsules produites est plus proche de 600-800 μm que de 400 μm) : voir Zimmermann, « Fabrication of homogeneously cross-linked, functional alginate microcapsules validated by NMR-, CLSM-, and AFM-imaging », Biomaterials, 2003. 24: p. 2083-2096 ;
- une différence de potentiel, qui est la technique d'encapsulation la plus utilisée lorsque la priorité est de diminuer la taille des capsules (la taille des capsules varie dans ce cas entre 200 et 800 μm) ; voir Goosen, « Electrostatic droplet generation for encapsulation of somatic tissue: assessment of high-voltage power supply », Biotechnol. Prog., 1997. 13 (497-502) ; ou

- une technique de vibration, qui présente l'inconvénient d'être parfois limitée par les viscosités des solutions utilisées ; voir Seifert, « Production of small, monodispersed alginate beads for cell immobilization » Biotechnol.Prog., 1997. 13: p. 562-568.

Ces techniques connues de l'art antérieur comportent un certain nombre d'inconvénients :

- la taille des capsules n'est pas forcement adaptée a la taille des cellules/ilots à encapsuler ;
- la dispersion en taille des capsules augmente lorsque la taille des gouttes diminue ;
- les capsules produites ne sont pas forcement sphériques, ce qui entraîne un manque de reproductibilité.

Outre ces problèmes, la plupart des techniques actuelles d'encapsulation n'offrent aucun moyen de contrôle sur le nombre de cellules ou d'amas contenus dans chaque goutte. En effet, le nombre de cellules/amas encapsulés n'est assuré que statistiquement en ajustant la concentration de la suspension de cellules (ou d'amas) dans la solution de polymère servant de matrice d'encapsulation (cette concentration dépend de la taille des particules à encapsuler et de la taille souhaitée des capsules). Les systèmes classiques produisent donc un très grand nombre de capsules vides et de capsules contenant un nombre variable de cellules/amas.

Les capsules vides ont pour principal inconvénient d'augmenter le volume total de capsules à transplanter et d'empêcher leur transplantation dans des zones qui seraient particulièrement propices a la revascularisation des tissus, essentielle pour éviter la nécrose des cellules encapsulées, qui doivent être a proximité du réseau sanguin pour être bien approvisionnées en nutriments et en oxygène. Par exemple, pour le traitement du diabète de type 1, une diminution du volume total de capsules à transplanter permettrait d'implanter les ilots encapsules dans le foie ou la rate, régions plus favorables à la revascularisation que la cavité péritonéale où les capsules sont classiquement implantées pour des questions d'encombrement stérique.

Lorsque les capsules contiennent un nombre variable de cellules/amas, il existe un risque de protubérance de ces cellules/amas à la surface de la capsule, ce qui est susceptible de déclencher une réaction immunitaire de rejet de la greffe. Ce problème est d'autant plus important lorsque la taille de la capsule est ajustée à la taille de l'objet à encapsuler.

Pour résoudre les inconvénients des techniques conventionnelles mentionnées ci-dessus, des dispositifs et procédés d'encapsulation basés sur des techniques microfluidiques ont été proposés. Voir par exemple :

- Workman et al. « Microfluidic chip-based synthesis of alginate microspheres for encapsulation of immortalized human cells » Biomicrofluidics, 2007. 1. Cet article décrit un procédé de fabrication de capsules de diamètre compris entre 80 et 400 μm environ au moyen d'un effet dit de « focalisation hydrodynamique ».
- Sugiura et al. « Size control of calcium alginate beads containing living cells using micronozzle array », Biomaterials, 2005. 26: p. 3327-3331. Dans le procédé décrit dans cette publication, des capsules de taille comprise entre 50 et 200 μm sont formées par injection d'alginate dans une phase huileuse à travers des « microbuses » d'un diamètre de 30 μm.

Ces techniques permettent un meilleur contrôle de la taille des capsules, qui est par ailleurs moindre que dans le cas des techniques conventionnelles; mais le nombre de particules (cellules ou amas) par capsule reste très variable.

A ce jour, la seule technique connue des inventeurs permettant l'encapsulation de particules isolées repose sur l'utilisation conjointe de la microfluidique et de pinces optiques permettant de déplacer une cellule contenue dans la phase aqueuse vers une interface eau/huile au niveau d'une jonction en T. Cette technique présente de plus l'avantage de produire une capsule du même ordre de grandeur que la taille de la cellule encapsulée. Cependant, elle ne permet pas d'atteindre des débits d'encapsulation suffisants pour rendre son application envisageable en dehors du domaine de la recherche scientifique.

L'invention vise à résoudre au moins certains des inconvénients de l'art antérieur.

En particulier, l'invention vise à permettre l'encapsulation d'un nombre contrôlé et reproductible de cellules ou amas de cellules. Avantageusement, l'invention permet la fabrication de capsules dont la taille est bien adaptée au nombre ce cellules/amas contenus, et évite la production de capsules vides.

Le dispositif de l'invention est de type microfluidique, présente une structure simple et peut être réalisé à bas coût par des techniques classiques de microfabrication.

Plus précisément un objet de l'invention est un dispositif microfluidique pour l'encapsulation contrôlée de particules de dimensions sub-millimétriques, ou d'amas de telles particules, comportant : un premier conduit microfluidique d'amenée d'une première phase liquide contenant en suspension des particules ou amas de particules à encapsuler ; un deuxième conduit microfluidique de circulation d'une deuxième phase liquide non miscible avec ladite première phase liquide ; le premier conduit débouchant dans le deuxième conduit et formant une jonction fluidique avec ce dernier ; au moins un conduit microfluidique d'évacuation de la première phase liquide circulant dans ledit premier conduit, pourvu d'une embouchure disposée en amont de ladite jonction et susceptible d'être bouchée, au moins partiellement, par une particule ou amas de particules en suspension, provoquant ainsi une surpression dans le premier conduit ; moyennant quoi des particules ou des amas de particules entraînés par ladite suspension le long dudit premier conduit sont susceptibles de s'accumuler au niveau de ladite jonction, piégés au niveau d'une interface entre les deux phases liquides non miscibles, jusqu'à être injectés dans ledit deuxième conduit par la surpression provoquée par l'obstruction de la ou des embouchures dudit ou desdits conduits d'évacuation.

Avantageusement, le dispositif de l'invention peut comporter au moins une paire de conduits microfluidiques d'évacuation disposés de part et d'autre dudit premier conduit.

En particulier le dispositif peut ne comporter qu'un seul conduit ou paire de conduits d'évacuation.

Selon un premier mode de réalisation, la section de l'embouchure dudit ou desdits conduits d'évacuation et sa distance de la jonction, ainsi que la section du premier conduit, peuvent être choisis de telle manière que l'arrivée d'une seule particule ou amas de particules au niveau de ladite jonction induit dans le premier conduit une surpression suffisante pour permettre l'injection de ladite particule ou amas de particules dans le deuxième conduit.

Selon un deuxième mode de réalisation, la section de l'embouchure dudit ou desdits conduits d'évacuation et sa distance de la jonction, ainsi que la section du premier conduit, peuvent être choisis de telle manière qu'une surpression suffisante pour permettre l'injection d'une particule ou amas de particules dans le deuxième conduit n'est atteinte que lorsqu'un nombre prédéterminé de particules ou amas de particules, supérieur à un et de préférence compris entre 2 et 10, est piégé au niveau de la jonction par ladite interface entre les deux phases liquides non miscibles.

Selon un troisième mode de réalisation le dispositif peut comporter au moins deux conduits ou paires de conduits d'évacuation dont les embouchures sont situées à des distances différentes de ladite jonction, dans lequel la section de l'embouchure dudit ou desdits conduits d'évacuation et sa distance de la jonction, ainsi que la section du premier conduit, sont choisis de telle manière qu'une surpression suffisante pour permettre l'injection d'une particule ou amas de particules dans le deuxième conduit n'est atteinte que lorsque les deux conduits ou paires de conduits sont obstrués, moyennant quoi l'injection simultanée d'une pluralité de particules ou amas de particules peut être obtenue.

Selon un troisième mode de réalisation le dispositif peut comporter un réseau de micro-piliers au niveau de ladite ou de chaque embouchure dudit ou desdits conduits d'évacuation.

Le dispositif peut comporter également un réseau de micro-piliers au niveau de la jonction fluidique entre le premier et le deuxième conduit.

Ladite jonction fluidique peut être une jonction en « T », le premier et le deuxième conduit formant entre eux un angle compris entre 60° et 90°, de préférence entre 80° et 90° et d'une manière encore préférée sensiblement égal à 90°.

En variante, ladite jonction fluidique peut être une jonction de focalisation à flux microfluidique.

Selon différentes variantes de réalisation de l'invention :

- Lesdits premier et deuxième conduits peuvent présenter des sections comprises entre 110 à 130% du diamètre de l'objet à encapsuler ; ledit ou lesdits conduits d'évacuation des sections de l'ordre de 30 à 60% de la section du premier conduit; et les embouchures dudit ou desdits conduits ou paires de conduits d'évacuation de ladite jonction peuvent être comprises entre 10 à 50% du diamètre de l'objet à encapsuler.
- Lesdits conduits peuvent être constitués par des sillons gravés ou moulés dans un substrat.
- Alternativement, le dispositif peut présenter une structure tridimensionnelle et être constitué par un empilement de substrats planaires moulés ou gravés, dans lequel le premier conduit traverse de manière sensiblement perpendiculaire lesdits substrats, tandis que le deuxième conduit et le ou les conduits d'évacuation sont formés au niveau des interfaces entre substrats et parallèlement à ces derniers.
- Les dimensions desdits conduits peuvent être adaptées pour permettre l'encapsulation de cellules ou bactéries.

Un autre objet de l'invention est un système microfluidique pour l'encapsulation contrôlée de particules de dimensions sub-millimétriques ou des amas de telles particules, comportant : un dispositif tel que décrit ci-dessus ; des moyens d'injection, dans le premier conduit dudit dispositif, d'une première phase liquide contenant en suspension des particules de dimensions sub-millimétriques ou des amas de telles particules à encapsuler, ladite phase liquide contenant également au moins un agent d'encapsulation ; des moyens d'évacuation de la première phase liquide circulant dans ledit premier conduit à travers ledit ou lesdits conduits d'évacuation ; et des moyens d'injection, dans le deuxième conduit dudit dispositif, d'une deuxième phase liquide non miscible avec ladite première phase liquide ; dans lequel lesdits moyens d'injection de la première et de la deuxième phase liquide, ainsi que lesdits moyens d'évacuation de la première phase liquide sont adaptés pour permettre la formation d'une interface stable entre les deux phases liquides non miscibles au niveau de ladite jonction, ainsi que la rupture de ladite interface en cas d'obstruction d'un ou plusieurs desdits conduits d'évacuation.

Un tel système peut comporter également une unité de gélification reliée audit deuxième conduit en aval de ladite jonction, ladite unité étant adaptée pour : recevoir en entrée ladite deuxième phase liquide contenant en suspension les particules ou amas de particules injectés dans ledit deuxième conduit, entourés d'une pellicule formée par la première phase liquide ; et provoquer la gélification de l'agent d'encapsulation contenu dans ladite pellicule de manière à constituer des capsules contenant lesdites particules ou amas de particules.

Encore un autre objet de l'invention est un procédé microfluidique pour l'encapsulation contrôlée de particules sub-millimétriques ou d'amas de telles particules, comportant les étapes consistant à : injecter, dans le premier conduit d'un dispositif tel que décrit plus haut, une première phase liquide contenant en suspension des particules ou amas de particules à encapsuler, ladite phase liquide contenant également au moins un agent d'encapsulation ; évacuer la phase liquide circulant dans ledit premier conduit à travers ledit ou lesdits conduits d'évacuation ; et injecter, dans le deuxième conduit dudit dispositif, une deuxième phase liquide non miscible avec ladite première phase liquide ; les pressions d'injection de la première et de la deuxième phase liquide, ainsi que le débit d'évacuation de la première phase liquide, étant choisis de manière à permettre la formation d'une interface stable entre les deux phases liquides non miscibles au niveau de ladite jonction ainsi que la rupture de ladite interface et l'injection dans ledit deuxième conduit d'un nombre contrôlé de particules ou amas de particules en cas d'obstruction d'un ou plusieurs desdits conduits d'évacuation.

Le procédé peut comporter également une étape de gélification de l'agent d'encapsulation contenu dans une pellicule constituée de ladite première phase liquide entourant les particules ou amas de particules injectées dans ledit deuxième conduit afin de former des capsules à paroi solide enfermant lesdites particules ou amas de particules.

Lesdites particules peuvent être choisies parmi des cellules, en particulier humaines, et des bactéries. En particulier, il peut s'agir de cellules pancréatiques, telles que les cellules β, ou plus généralement de cellules secrétant des hormones, protéines ou toute autre substance d'intérêt thérapeutique. Il peut également s'agir de cellules souches. Les amas de particules peuvent être, par exemple, des îlots de Langerhans.

D'autres caractéristiques, détails et avantages de l'invention ressortiront à la lecture de la description faite en référence aux dessins annexés donnés à titre d'exemple et qui représentent, respectivement :

- les figures 1 à 3 et 8, des schémas de principe de quatre différents modes de réalisation d'un dispositif selon l'invention ;
- les figures 4a à 4e, la distribution de la pression hydraulique dans le dispositif de la figure 1 ;
- la figure 5, un quatrième mode de réalisation du dispositif de l'invention ;
- la figure 6, un cinquième mode de réalisation du dispositif de l'invention ; et

- la figure 7, un schéma d'un système complet d'encapsulation comprenant un dispositif selon l'une des figures 1 à 3, 5 ou 6.

Le dispositif microfluidique de l'invention utilise deux phases non miscibles : par exemple une première phase aqueuse, indiquée par $\phi_1$ sur les figures, et une deuxième phase $\phi_2$ de type huileux. La première phase peut être par exemple constituée par un tampon biologique ou un sérum physiologique ; elle contient en suspension les cellules ou amas de cellules (C sur les figures) à encapsuler et, en solution, un agent d'encapsulation tel qu'un hydrogel d'alginate, du chitosan, des carraghenanes, des gels d'agarose, polyéthylène glycol (PEG) ou tout autre polymère adapté.

La deuxième phase peut être une huile végétale (par exemple huile de tournesol), une huile minérale, silicone, un solvant perfluoré, etc.

En fait, il n'est pas réellement essentiel que la première phase soit de type aqueux et la deuxième de type huileux. Ce qui est essentiel est que ces deux phases soient non miscibles et, sauf applications particulières, que la première phase permette la survie des cellules ou amas de cellules.

Le dispositif comporte un premier et un deuxième conduits microfluidiques principaux, indiqués par les références 10 et 20, qui forment une jonction « en T » 30. Des conduits secondaires 40, 40', 50, 50' se départent du premier conduit 10, à proximité de la jonction 30.

Le premier conduit 10, alimenté en phase aqueuse $\phi_1$, débouche dans le deuxième conduit 20, alimenté en phase huileuse $\phi_2$ ; les deux conduits forment ainsi une jonction fluidique « en T ». L'expression « jonction en T », connue dans le domaine de la microfluidique, ne doit pas être interprétée trop strictement ; en effet, il n'est pas indispensable que les deux conduits forment entre eux un angle exactement égal à 90°. L'angle peut, par exemple, être compris entre 60° et 90° et de préférence entre 80° et 90°, les valeurs plus proches à 90° étant particulièrement préférées.

Les deux phases liquides $\phi_1$ et $\phi_2$ n'étant pas miscibles, une interface I se forme au niveau de la jonction 30. La phase aqueuse $\phi_1$, empêchée par ladite interface de s'écouler dans le deuxième conduit, s'évacue à travers les conduits secondaires 40, 40', 50, 50'. Les pressions et débits des deux phases liquides sont dimensionnés de telle manière que l'interface I reste stable par équilibre entre lesdites pressions et la tension interfaciale.

Les conduits secondaires, ou d'évacuation 40, 40', 50, 50' présentent une section à l'embouchure dans le canal 10 moindre que les conduits principaux 10 et 20, et ne permettent pas la circulation des cellules C. Les particules (cellules ou amas), amenées par la première phase liquide $\phi_1$, restent donc piégées au niveau de l'interface I, alors que ladite phase liquide s'évacue par les conduits secondaires. Cela continue jusqu'à ce que lesdites particules viennent obstruer les embouchures desdits conduits secondaires ; cela entraîne une surpression entraînant la rupture de l'interface I et l'injection d'une ou plusieurs particules dans le deuxième conduit.

Plus précisément, ce qui est injecté dans le deuxième conduit par la surpression transitoire engendrée par le bouchage des conduits d'évacuation est une gouttelette de première phase liquide, contenant une ou plusieurs particules. La taille de cette gouttelette, indiquée par K dans les figures, est adaptée à celle des cellules ou amas qu'elle contient : en fait, on peut voir la gouttelette K comme une fine pellicule liquide entourant une ou plusieurs particules. Cette pellicule liquide constitue le précurseur de la capsule qui sera formé, d'une manière connue par elle-même, par gélification de l'agent d'encapsulation (par exemple, l'alginate) en solution dans la première phase liquide.

La formation de gouttelettes dans une phase liquide non miscible au moyen d'une jonction en T entre deux conduits microfluidiques est connue de l'art antérieur. Voir par exemple l'article de T. Thorsen et al. « Dynamic pattern formation in a vesicle-generating microfluidic device » Physical Review Letters, 2001. 86 (18): p. 4163-4166.

Le dispositif de l'invention permet de contrôler précisément le nombre de particules (cellules ou amas) qui sont injectées en même temps, et qui sont donc contenues dans une même capsule. Cela sera compris à l'aide de trois exemples.

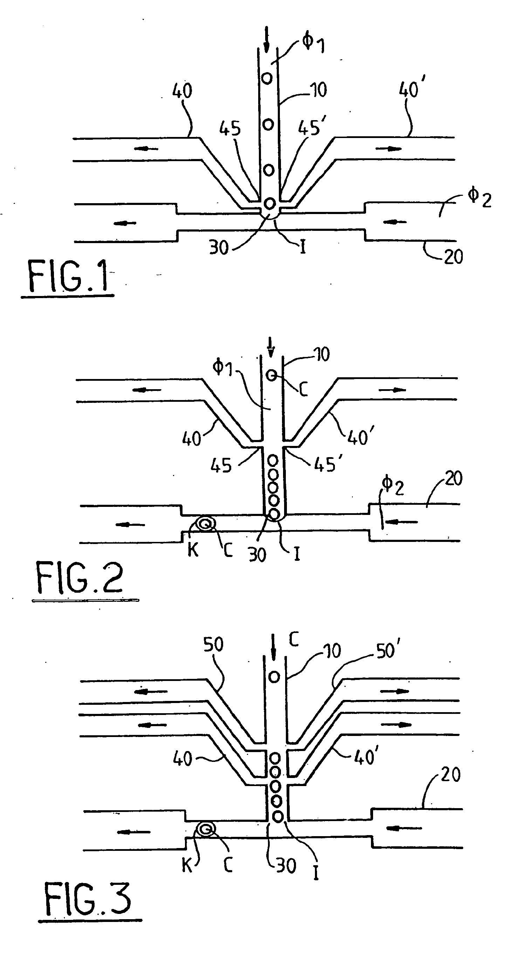

Dans l'exemple de la figure 1, deux conduits secondaires d'évacuation 40, 40' sont prévus symétriquement de part et d'autre du premier conduit 10 à proximité immédiate de la jonction 30. La section des embouchures 45, 45' desdits conduits d'évacuation 40, 40' et leur distance de la jonction 30, ainsi que la section du premier conduit, sont choisis de telle manière que l'arrivée d'une seule particule au niveau de ladite jonction suffit pour obstruer les conduits d'évacuation et induire ainsi une surpression assez importante pour permettre l'injection de ladite particule dans le deuxième conduit. Autrement dit, les particules sont injectées une par une, dès qu'elles arrivent au niveau de l'interface I. Bien entendu, pour cela il faut que les particules soient suffisamment dispersées dans la première phase liquide pour pouvoir arriver à la jonction de manière individuelle, et non groupées.

La figure 1 est une représentation bidimensionnelle du dispositif, mais en réalité les conduits présentent une profondeur finie. Cette profondeur doit naturellement être prise en compte pour le dimensionnement des sections desdits conduits.

Les profondeurs des différents conduits du dispositif peuvent être uniformes ou différenciées. En règle générale, la partie terminale du premier conduit 10 devra être adaptée au diamètre des particules à encapsuler pour permettre l'obstruction des conduits d'évacuation 40, 40' ; au contraire, le deuxième conduit 20 et le conduit d'amené des particules situé en amont dudit conduit 10 (non représenté) peuvent présenter une profondeur plus importante afin de réduire les pertes de charges et limiter les cisaillements sur les particules. Cela est particulièrement avantageux dans le cas de particules de très petites dimensions (micrométriques, voire sub-micrométriques) telles que des bactéries.

Dans l'exemple de la figure 2, les conduits d'évacuation 40, 40' (ou, plus précisément, leur embouchures 45, 45') sont situées à une distance plus importante de la jonction 30. De cette manière, pour obstruer lesdits conduits d'évacuation, il faut que plusieurs particules C soient piégées au niveau de l'interface I. La figure illustre un exemple dans lequel cinq particules sont déjà empilées dans le premier conduit 10, et maintenues dans ledit conduit 10 par la tension de surface à l'interface $\phi_1$-$\phi_2$, sans que les conduits secondaires 40, 40' soient obstrués. Par contre, l'arrivée d'une sixième particule provoquera une obstruction, et donc une surpression dans le premier conduit, qui à son tour causera l'injection dans le deuxième conduit de la première particule de la « queue ». Dans ce cas aussi, les particules sont injectées une par une.

L'avantage de ce mode de réalisation, par rapport à celui de la figure 1, est qu'il est plus simple à réaliser d'un point de vue technologique, surtout lorsqu'on veut encapsuler des cellules isolées ou des amas de petites dimensions : en effet, dans ces cas, la mise en oeuvre du premier mode de réalisation nécessiterait la fabrication de conduits secondaires très près de la jonction 30.

Un dispositif du type représenté sur la figure 2 peut également convenir à l'injection simultanée de plusieurs particules : tout dépend du dimensionnement des conduits et des pressions d'injection (et éventuellement d'évacuation) des deux phases liquides.

Ce deuxième mode de réalisation est également mieux adapté lorsque les particules à injecter sont polydisperses. Cependant, dans ce cas, le contrôle du nombre de particules contenues dans chaque capsule sera moins précis que lorsque la suspension de particules est monodisperse, mais la taille des capsules restera monodisperse, et si formation de capsule il y a, elles contiendront forcément des cellules et ne seront pas vides.

En outre, ce deuxième mode de réalisation permet d'utiliser un conduit principal relativement large même pour l'encapsulation de particule de petites dimensions, ce qui réduit la pression de Laplace et donc les forces de compression, potentiellement nuisibles, qui s'exercent sur lesdites particules.

Comme cela a été évoqué plus haut, la distance entre les embouchures 45, 45' de conduits d'évacuation et la jonction 30 n'est pas le seul paramètre à prendre en considération : la section desdites embouchure et du premier conduit doivent également être dimensionnés de manière appropriée pour assurer qu'une obstruction suffisamment complète des conduits d'évacuation ne se produise qu'à l'arrivée de la (N+1)ème particule, avec N déterminée précisément en phase de conception du dispositif. Ce dimensionnement est effectué en supposant connues les pressions d'injection et les tensions de surface des deux phases liquides, ainsi que la taille des particules à encapsuler.

Dans un troisième mode de réalisation, représenté sur la figure 3, deux paires de conduits d'évacuation (40, 40' et 50, 50') sont prévues à des distances différentes de la jonction 30. Le dispositif est dimensionné pour que l'obstruction d'une seule paire de conduits ne suffise pas pour provoquer la rupture de l'interface fluide I. Par exemple, comme représenté sur la figure, la première paire de conduits 40, 40' (celle plus proche de la jonction) est obstruée par l'accumulation de quatre particules C au niveau de la jonction, tandis que la deuxième paire de conduits 50, 50' n'est obstruée que par l'arrivée d'une sixième cellule.

Lorsque ladite sixième cellule arrive, la surpression dans le premier conduit atteint un niveau suffisant à permettre la rupture de l'interface I, et les particules C commencent à être injectées dans le deuxième conduit 20. Les embouchures des deuxièmes conduits d'évacuation sont ainsi dégagées, mais celles des premiers conduits restent obstruées. Ainsi, une surpression subsiste dans le conduit 10 ; cette surpression est insuffisante, à elle seule, pour rompre l'interface fluide I, mais est suffisamment importante pour entretenir une rupture déjà amorcée. Ladite interface ne peut donc se reformer que lorsque les embouchures de tous les conduits d'évacuation ont été dégagées, c'est à dire après l'injection dans le conduit 20 de trois particules.

Ce troisième mode de réalisation convient donc à l'encapsulation simultanée d'un nombre contrôlé, supérieur à 1 et de préférence compris entre 1 et 10, de cellules ou amas de cellules.

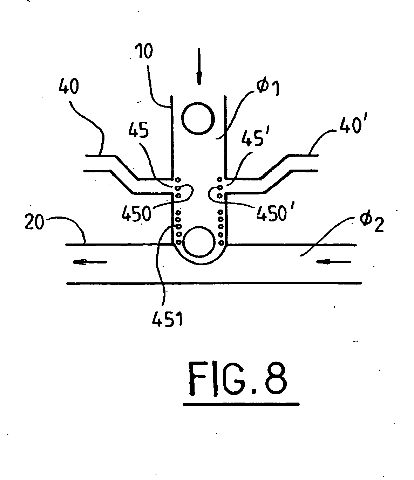

Dans un quatrième mode de réalisation, représenté sur la figure 8, des réseaux de micro-piliers 450, 450' sont prévus au niveau des embouchures 45, 45' des conduits d'évacuation 40, 40'. En l'absence de cellules, le débit de la phase $\phi_1$ filtre à travers les interstices entre piliers et s'évacue dans les deux conduits latéraux d'évacuation 40 et 40'. Lorsqu'une ou plusieurs cellules viennent s'immobiliser au voisinage de l'interface avec la phase $\phi_2$, la résistance hydraulique de la phase $\phi_1$ est brusquement accrue et une pression est exercée sur la ou les cellules immobilisées. Par un dimensionnement adéquat des pertes de charge à travers les piliers et dans les conduits d'évacuation, cette surpression est suffisante pour éjecter dans la phase $\phi_2$, une ou plusieurs cellules emprisonnées dans des gouttes. L'intérêt des piliers réside dans un dosage précis des pertes de charge et également dans un piégeage contrôlé des cellules entre les piliers. En effet, comme les cellules sont légèrement déformables, elles viennent se loger dans l'intervalle entre les piliers, et sont donc précisément localisées. Si $D$ désigne le diamètre des cellules à encapsuler, le diamètre des piliers sera de préférence compris entre $D/5$ et $D/3$, l'espacement entre deux piliers, selon la direction du canal 10, étant de préférence compris entre $\dfrac{2D}{3}$ et $\dfrac{4D}{5}$.

Comme le montre la figure 8, un réseau additionnel de micro-piliers 451 peut également être prévu au niveau de la jonction 30 entre les conduits 10 et 20 pour stabiliser et immobiliser les cellules de façon très précise. Ce réseau additionnel 451 peut également être utilisé indépendamment des réseaux 405, 450' disposés au niveau des embouchures des conduits d'évacuation.

Dans les quatre modes de réalisation représentés sur les figures 1 à 3 et 8, les conduits d'évacuation sont disposés par paires, de manière exactement symétrique par rapport au premier conduit 10. Cela n'est pas réellement essentiel, et des conduits d'évacuation asymétriques, voire disposés d'un seul côté du premier conduit peuvent être utilisés. Cependant, une disposition au moins approximativement symétrique des conduits d'évacuation est préférable pour éviter que les particules circulant dans le premier conduit ne soient aspirées par des conduits d'évacuation disposées d'un seul côté, obstruant ces derniers de manière intempestive.

Les dimensions physiques d'un dispositif tel que décrit ci-dessus dépendent de l'application envisagée, et en particulier de la taille des particules à encapsuler. A titre d'exemple, des cellules isolées ont des diamètres de l'ordre de quelques micromètres, tandis que des amas du type des îlots de Langerhans peuvent atteindre voire dépasser les 500 $\mu$m de diamètre. En règle générale, cependant, le dispositif doit rester de type microfluidique : cela signifie que les dimensions transversales des conduits doivent être comprises entre quelques micromètres ou dizaines de micromètres à un millimètre ou tout au plus quelques millimètres.

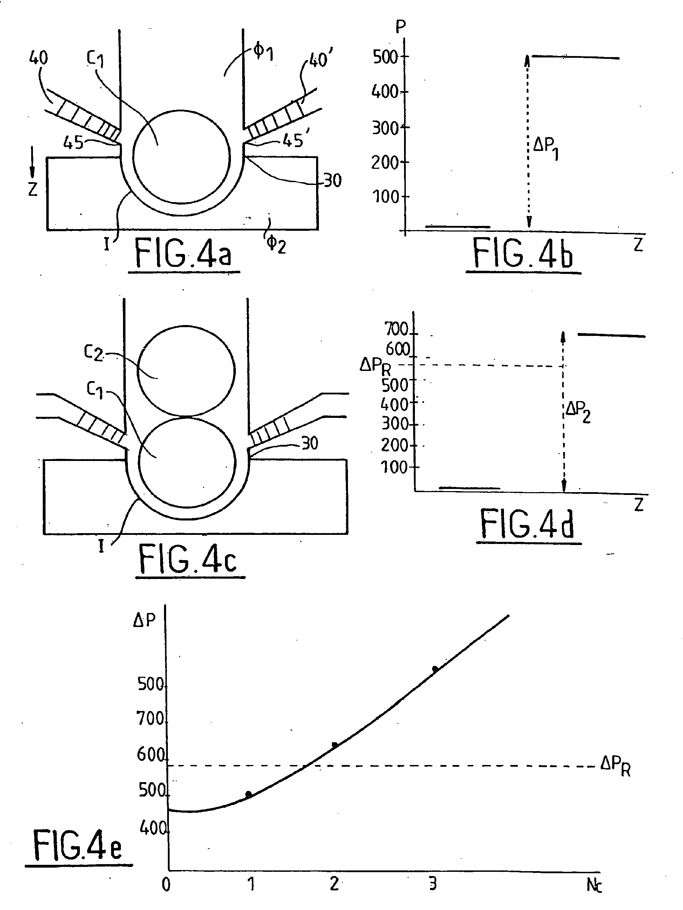

Les figures 4a et 4c montent un agrandissement de la région immédiatement adjacente à la jonction 30 d'un dispositif selon l'invention. Les niveaux de gris représentent les valeurs de pression dans les deux phases liquides $\phi_1$ et $\phi_2$. Les graphiques 4b et 4d illustrent le saut de pression $\Delta P_1$, $\Delta P_2$ à travers l'interface I, pour le cas de la figure 4a et 4c respectivement.

Dans le cas des figures 4a et 4b, une seule particule C1 est présente à l'interface I entre les deux phases liquides. Les embouchures des conduits d'évacuation sont situées à une distance de la jonction qui est inférieure au diamètre de cette particule ; cependant ; lesdits conduits d'évacuation ne sont que partiellement obstrués, car il reste sur les côtés du conduit principal un espace suffisant pour permettre l'écoulement de la première phase liquide. Cet exemple montre clairement que la distance embouchures - jonction n'est qu'un des paramètres à prendre en compte lors du dimensionnement du dispositif.

L'arrivée d'une deuxième particule C2 (figure 4c) constitue un obstacle supplémentaire à l'évacuation du liquide ; par conséquent, le saut de pression à l'interface augmente et atteint une valeur $\Delta P_2$ supérieure à la valeur critique $\Delta P_R$ qui conduit à la rupture de l'interface I. Au moins l'une des deux particules sera donc injectée dans le deuxième conduit 20. Plus exactement : les deux particules seront injectées si la valeur de $\Delta P_1$ est suffisante à entretenir la rupture de l'interface ; autrement, seule la particule C1 (la première de la file) sera injectée, une interface stable se reformant immédiatement après.

La figure 4e illustre la dépendance de la valeur du saut de pression $\Delta P$ par rapport au nombre de particules $N_C$ à l'interface I ; la ligne continue n'est qu'un guide pour l'oeil. On remarque que $\Delta P$ est différent de zéro même lorsqu'il n'y a pas de particules à la jonction : cette valeur $\Delta P(0)$ est égale à la tension de surface à l'interface.

La différence de pression $\Delta P_R$ qui provoque la rupture de l'interface vaut $\Delta P_R = \gamma_{12}\left(\dfrac{1}{w}+\dfrac{1}{d}\right)$ où $\gamma_{12}$ est la tension interfaciale entre les deux phases liquides, tandis que w et d sont respectivement la largeur et la profondeur du premier conduit, supposé à section rectangulaire.

Les valeurs de pression sur les graphiques 4b, 4d et 4e sont données en Pascal (Pa).

Les modes de réalisation des figures 1 à 3 se rapportent à un dispositif à géométrie planaire, de type puce microfluidique.

Un tel dispositif peut être fabriqué à partir d'un substrat en silicium, silice ou verre par des techniques classiques de photolithographie et gravure empruntées à la microélectronique. Cette technologie sur silicium a l'avantage d'être très précise (de l'ordre du micromètre) et peu limitative en ce qui concerne tant les profondeurs de gravure que les largeurs des motifs.

En variante, le dispositif peut être réalisé en matière plastique (notamment en polydimethylsiloxane - PDMS) par moulage, à partir d'un « master », réalisé à son tour par photolithographie.

En règle générale, de nombreux matériaux différents peuvent convenir à la réalisation d'un dispositif selon l'invention. De préférence, ces matériaux doivent pouvoir supporter une stérilisation, notamment si le dispositif est destiné à fabriquer des capsules implantables.

Le dispositif peut également être réalisé sous une forme tridimensionnelle, dans laquelle les entrées et sorties microfluidiques ne s'effectuent pas dans un même plan. Dans ce cas, la formation des capsules sera non seulement due à la surpression locale induite par l'obstruction des conduits secondaires d'évacuation, mais aussi a la force de sédimentation des cellules ou amas de cellules due à la force de pesanteur.

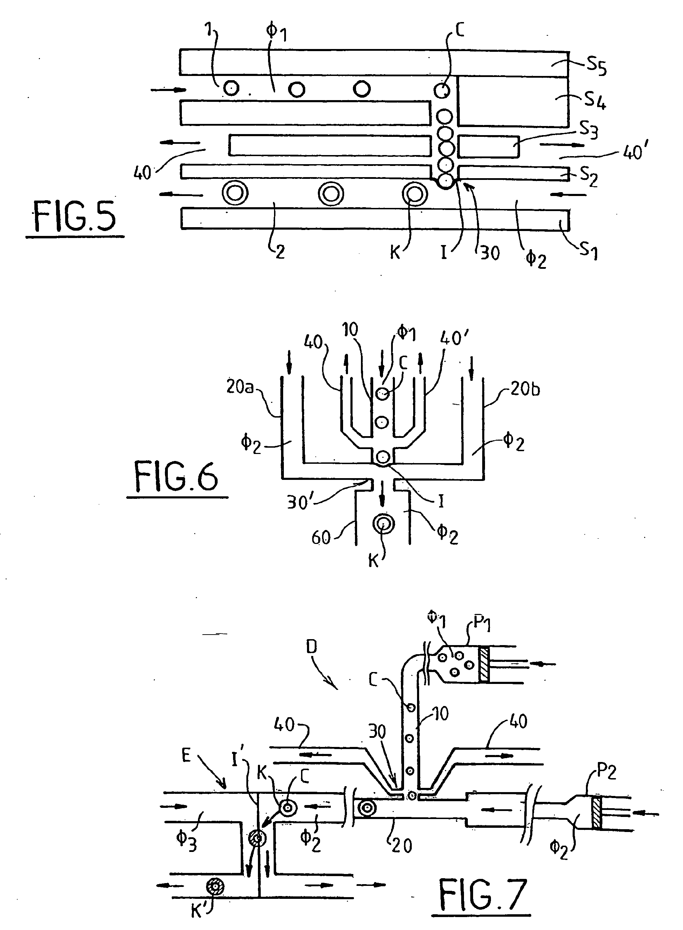

Un tel dispositif, dont un exemple de réalisation est représenté de manière simplifiée sur la figure 5, est constitué par un empilement de substrats planaires S1 - S5 comportant des motifs gravés ou moulés. Le deuxième conduit 20 et le ou les conduits d'évacuation 40, 40' sont formés par lesdits motifs gravés ou moulés au niveau des interfaces entre substrats et parallèlement à ces derniers. Au contraire, le premier conduit 10 est constitué par un empilement de trous passants, et traverse donc lesdits substrats de manière sensiblement perpendiculaire.

Dans les modes de réalisation des figures 1 à 5, le premier et le deuxième conduit forment entre eux une jonction fluidique 30 de type « en T » (au sens large, car il n'est pas essentiel que les deux conduits soient orthogonaux entre eux). Cela n'est cependant pas le seul cas possible. Ainsi, la figure 6 montre un dispositif selon un mode de réalisation alternatif de l'invention, basé sur l'utilisation d'une jonction à focalisation par flux microfluidique 30'.

Dans ce dispositif, le deuxième conduit est constitué par deux branches 20a, 20b, qui se rejoignent au niveau de la jonction 30'. Dans les deux branches, la deuxième phase liquide $\phi_2$ circule en direction de ladite jonction.

La jonction fluidique 30' n'est pas à trois branches, comme la jonction en T 30 des figures 1 à 3, mais à quatre branches : en effet, un troisième conduit 60 est prévu en regard du premier conduit 10 pour recevoir la phase huileuse en provenance des branches 20a, 20b et les particules C injectées dans la jonction en provenance de ce dernier.

Le principe de la focalisation par flux microfluidique (ou MFF, microfluidic flow focusing) est connu depuis l'article de S.L. Anna, N. Bontoux, et H.A. Stone, « Formation of dispersions using "flow focusing" in microchannels », Applied Physics Letters, 2003. 82(3): pages 364-366

La figure 7 représente schématiquement un système microfluidique complet d'encapsulation de cellules ou amas de cellules. Un tel système comporte :

- un dispositif D selon l'invention ;
- un moyen d'injection, dans le premier conduit 10 dudit dispositif, d'une suspension formée par la première phase liquide $\phi_1$ et par les particules à encapsuler ; il peut s'agir par exemple d'une seringue avec pousse-seringue P1 ou d'un contrôleur en pression ;
- un moyen d'injection, dans le deuxième conduit 20 du dispositif, de la deuxième phase liquide $\phi_2$; il peut s'agir également d'une seringue avec pousse-seringue P2 ou d'un contrôleur en pression ;
- éventuellement, de moyens d'aspiration de ladite première phase liquide par les conduits d'évacuation 40, 40' (non représentés ; en variante, l'évacuation du liquide peut se faire à l'air libre) ;
- d'un moyen microfluidique E de gélification des capsules ; sur la figure, la référence K indique les « capsules » non encore gélifiées, constituées simplement des cellules ou amas de cellules entourées d'une pellicule de première phase liquide, tandis que la référence K' indique les capsules gélifiées.

Le moyen microfluidique E peut être un dispositif microfluidique en forme de H dans lequel la deuxième phase liquide $\phi_2$ rentre en contact avec une troisième phase liquide non miscible $\phi_3$ contenant en solution des agents de gélification (par exemple, des ions $Ca^+$ dans le cas d'une encapsulation avec de l'alginate). Les capsules K traversent l'interface I' entre la deuxième et la troisième phase liquide, subissent une réaction de gélification et sont récupérées pour être implantées dans un patient. Un tel dispositif est décrit dans le document US 2006/0051329. Le document WO 2005/103106 décrit un autre dispositif microfluidique de gélification pouvant être utilisé en combinaison avec le dispositif d'encapsulation de l'invention.

Le dispositif d'encapsulation D et le dispositif de gélification E peuvent être réalisés en forme intégrée (sur un même substrat ou empilement de substrats) ou discrète.

On remarque que le système de la figure 7 ne doit pas nécessairement comporter une unité de tri des capsules : cela est dû au fait que le dispositif D de l'invention permet d'obtenir des capsules de dimensions et de contenu très uniformes, et en particulier d'éviter la formation de capsules vides. Un dispositif de tri des particules ou amas à encapsuler peut, par contre, être prévu avantageusement en amont de l'unité d'encapsulation. Des très nombreux dispositifs microfluidiques de tri sont connus de l'art antérieur; voir par exemple les documents WO 2004/037374, WO 2006/102258, WO 2002/02316, ainsi que la Demande de brevet en France n° 0802575 « Système microfluidique et procédé pour le tri d'amas de cellules et de préférence pour leur encapsulation en continu suite à leur tri », au nom du présent déposant et déposée le 13 Mai 2008.

Le dispositif de gélification ne doit pas toujours être présent. S'il est généralement indispensable lorsqu'on souhaite obtenir des capsules à paroi solide pour implantation dans un patient, il y a des cas (notamment dans le domaine de la recherche ou des analyses biologiques) où il suffit de disposer de cellules ou amas de cellules « encapsulés » dans une gouttelette de liquide en suspension. Dans ce cas, la première phase liquide $\phi_1$ ne doit pas nécessairement contenir un agent d'encapsulation.

**Revendications**

1. Dispositif microfluidique (D) pour l'encapsulation contrôlée de particules de dimensions sub-millimétriques, ou d'amas de telles particules, comportant :

    un premier conduit microfluidique (10) d'amenée d'une première phase liquide ($\phi_1$) contenant en suspension des particules ou amas de particules (C) à encapsuler ;
    - un deuxième conduit microfluidique (20) de circulation d'une deuxième phase liquide ($\phi_2$) non

miscible avec ladite première phase liquide ; le premier conduit débouchant dans le deuxième conduit et formant une jonction fluidique (30, 30') avec ce dernier ;

- au moins un conduit microfluidique d'évacuation (40, 40', 50, 50') de la première phase liquide circulant dans ledit premier conduit, pourvu d'une embouchure (45, 45') disposée en amont de ladite jonction et susceptible d'être bouchée, au moins partiellement, par une particule ou amas de particules en suspension, provoquant ainsi une surpression dans le premier conduit ;

moyennant quoi des particules ou des amas de particules entraînés par ladite suspension le long dudit premier conduit sont susceptibles de s'accumuler au niveau de ladite jonction, piégés au niveau d'une interface (I) entre les deux phases liquides non miscibles, jusqu'à être injectés dans ledit deuxième conduit par la surpression provoquée par l'obstruction de la ou des embouchures dudit ou desdits conduits d'évacuation.

2. Dispositif selon la revendication 1, comportant au moins une paire de conduits microfluidiques d'évacuation disposés de part et d'autre dudit premier conduit.

3. Dispositif selon l'une des revendications précédentes, comportant un seul conduit ou paire de conduits d'évacuation

4. Dispositif selon la revendication 3, dans lequel la section de l'embouchure dudit ou desdits conduits d'évacuation et sa distance de la jonction, ainsi que la section du premier conduit, sont choisis de telle manière que l'arrivée d'une seule particule ou amas de particules au niveau de ladite jonction induit dans le premier conduit une surpression suffisante pour permettre l'injection de ladite particule ou amas de particules dans le deuxième conduit.

5. Dispositif selon la revendication 3, dans lequel la section de l'embouchure dudit ou desdits conduits d'évacuation et sa distance de la jonction, ainsi que la section du premier conduit, sont choisis de telle manière qu'une surpression suffisante pour permettre l'injection d'une particule ou amas de particules dans le deuxième conduit n'est atteinte que lorsqu'un nombre prédéterminé de particules ou amas de particules, supérieur à un et de préférence compris entre 2 et 10, est piégé au niveau de la jonction par ladite interface entre les deux phases liquides non miscibles

6. Dispositif selon l'une des revendications 1 ou 2 comportant au moins deux conduits ou paires de conduits d'évacuation dont les embouchures sont situées à des distances différentes de ladite jonction, dans lequel la section de l'embouchure dudit ou desdits conduits d'évacuation et sa distance de la jonction, ainsi que la section du premier conduit, sont choisis de telle manière qu'une surpression suffisante pour permettre l'injection d'une particule ou amas de particules dans le deuxième conduit n'est atteinte que lorsque les deux conduits ou paires de conduits sont obstrués, moyennant quoi l'injection simultanée d'une pluralité de particules ou amas de particules peut être obtenue.

7. Dispositif selon l'une des revendications précédentes, dans lequel ladite jonction fluidique (30) est une jonction en « T », le premier et le deuxième conduit formant entre eux un angle compris entre 60° et 90°, de préférence entre 80° et 90° et d'une manière encore préférée sensiblement égal à 90°.

8. Dispositif selon l'une des revendications 1 à 6, dans lequel ladite jonction fluidique (30') est une jonction de focalisation à flux microfluidique.

9. Dispositif selon l'une des revendications précédentes, dans lequel lesdits premier et deuxième conduits présentent des sections comprises entre 110 à 130% du d diamètre de l'objet à encapsuler ; ledit ou lesdits conduits d'évacuation des sections de l'ordre de 30 à 60% de la section du $1^{er}$ conduit; et les embouchures dudit ou desdits conduits ou paires de conduits d'évacuation de ladite jonction sont comprises entre 10 à 50% du diamètre de l'objet à encapsuler.

10. Dispositif selon l'une des revendications précédentes, comportant un réseau de micro-piliers (450, 450') au niveau de ladite ou de chaque embouchure dudit ou desdits conduits d'évacuation.

11. Dispositif selon l'une des revendications précédentes, comportant un réseau de micro-piliers (451) au niveau de la jonction fluidique entre le premier et le deuxième conduit.

12. Dispositif selon l'une des revendications précédentes présentant une structure planaire, dans lequel lesdits conduits sont constitués par des sillons gravés ou moulés dans un substrat.

13. Dispositif selon l'une des revendications 1 à 11 présentant une structure tridimensionnelle et étant constitué par un empilement de substrats ($S_1$ - $S_5$) planaires moulés ou gravés, dans lequel le premier conduit traverse de manière sensiblement perpendiculaire lesdits substrats, tandis que le deuxième conduit et le ou les conduits d'évacuation sont formés au niveau des interfaces entre substrats et parallèlement à ces derniers.

**14.** Dispositif selon l'une des revendications précédentes, dans lequel les dimensions desdits conduits sont adaptées pour permettre l'encapsulation de cellules ou bactéries.

**15.** Système microfluidique pour l'encapsulation contrôlée de particules de dimensions sub-millimétriques ou des amas de telles particules, comportant :

- un dispositif (D) selon l'une des revendications précédentes ;
- des moyens (P₁) d'injection, dans le premier conduit dudit dispositif, d'une première phase liquide contenant en suspension des particules de dimensions sub-millimétriques ou des amas de telles particules à encapsuler, ladite phase liquide contenant également au moins un agent d'encapsulation ;
- des moyens d'évacuation de la première phase liquide circulant dans ledit premier conduit à travers ledit ou lesdits conduits d'évacuation ; et
- des moyens d'injection (P₂), dans le deuxième conduit dudit dispositif, d'une deuxième phase liquide non miscible avec ladite première phase liquide ;

dans lequel lesdits moyens d'injection de la première et de la deuxième phase liquide, ainsi que lesdits moyens d'évacuation de la première phase liquide sont adaptés pour permettre la formation d'une interface stable entre les deux phases liquides non miscibles au niveau de ladite jonction, ainsi que la rupture de ladite interface en cas d'obstruction d'un ou plusieurs desdits conduits d'évacuation.

**16.** Système microfluidique selon la revendication 15 comportant également une unité (E) de gélification reliée audit deuxième conduit en aval de ladite jonction, ladite unité étant adaptée pour :

- recevoir en entrée ladite deuxième phase liquide contenant en suspension les particules ou amas de particules injectés dans ledit deuxième conduit, entourés d'une pellicule formée par la première phase liquide ; et
- provoquer la gélification de l'agent d'encapsulation contenu dans ladite pellicule de manière à constituer des capsules contenant lesdites particules ou amas de particules.

**17.** Procédé microfluidique pour l'encapsulation contrôlée de particules sub-millimétriques ou d'amas de telles particules, comportant les étapes consistant à :

- injecter, dans le premier conduit d'un dispositif selon l'une des revendications 1 à 14, une première phase liquide contenant en suspension des particules ou amas de particules à encapsuler, ladite phase liquide contenant également au moins un agent d'encapsulation ;
- évacuer la phase liquide circulant dans ledit premier conduit à travers ledit ou lesdits conduits d'évacuation ; et
- injecter, dans le deuxième conduit dudit dispositif, une deuxième phase liquide non miscible avec ladite première phase liquide ;

les pressions d'injection de la première et de la deuxième phase liquide, ainsi que le débit d'évacuation de la première phase liquide, étant choisis de manière à permettre la formation d'une interface stable entre les deux phases liquides non miscibles au niveau de ladite jonction ainsi que la rupture de ladite interface et l'injection dans ledit deuxième conduit d'un nombre contrôlé de particules ou amas de particules en cas d'obstruction d'un ou plusieurs desdits conduits d'évacuation.

**18.** Procédé selon la revendication 17, comportant également une étape de gélification de l'agent d'encapsulation contenu dans une pellicule constituée de ladite première phase liquide entourant les particules ou amas de particules injectées dans ledit deuxième conduit afin de former des capsules (K') à paroi solide enfermant lesdites particules ou amas de particules.

**19.** Procédé selon l'une des revendications 17 ou 18, dans lequel lesdites particules sont choisies parmi des cellules, en particulier humaines, et des bactéries.

**Claims**

**1.** A microfluidic device (D) for controlled encapsulation of particles of sub-millimetric dimensions, or clusters of such particles, the device comprising:

. a first microfluidic duct (10) for delivering a first liquid phase ($\phi_1$) containing in suspension particles or particles clusters (C) for encapsulating; a second microfluidic duct (20) for conveying a flow of a second liquid phase ($\phi_2$) that is immiscible with said first liquid phase; the first duct opening out into the second duct and forming a fluidic junction (30, 30') therewith; at least one discharge microfluidic duct (40, 40', 50, 50') for discharging the first liquid phase flowing in said first duct, being provided with a mouth (45, 45') disposed upstream from said junction and being suitable for being obstructed, at least in part, by a particle or particle cluster in suspension, thereby causing pressure to rise in the first duct;

whereby particles or particle clusters entrained by said suspension along said first duct are liable to accumulate at said junction, being trapped at an interface (I) between the two immiscible liquid phases, until they are injected into said second duct by the pressure rise caused by the mouth(s) of said discharge duct(s) being obstructed.

2. A device according to claim 1, including at least one pair of microfluidic discharge ducts disposed on either side of said first duct.

3. A device according to any preceding claim, including a single encapsulation duct or a single pair of encapsulation ducts.

4. A device according to claim 3, wherein the section of the mouth of said encapsulation duct(s) and the distance of the mouth(s) from the junction, and also the section of the first duct, are selected in such a manner that the arrival of a single particle or particle cluster at said junction induces in the first duct a pressure rise that is sufficient to enable said particle or particle cluster to be injected into the second duct.

5. A device according to claim 3, wherein the section of the mouth(s) of said discharge duct(s) and the distance of the mouth(s) from the junction, together with the section of the first duct, are selected in such a manner that a pressure sufficient for enabling a particle or particle cluster to be injected into the second duct is not reached until a predetermined number of particles or particle clusters is trapped at the junction by said interface between the two immiscible liquid phases, said predetermined number being greater than 1, and preferably lying in the range 2 to 10.

6. A device according to claim 1 or claim 2, including at least two discharge ducts or at least two pairs of discharge ducts having mouths situated at different distances from said junction, in which the section(s) of the mouths of said discharge ducts and the distance(s) of the mouths from the junction, and the section of the first duct, are selected in such a manner that a pressure sufficient for enabling a particle or particle cluster to be injected into the second duct is reached only when said at least two ducts or at least two pairs of ducts are obstructed, thereby making it possible to ensure that a plurality of particles or particle clusters are injected simultaneously.

7. A device according to any preceding claim, wherein said fluidic junction (30) is a T-junction, the first and second ducts forming between them an angle lying in the range 60° to 90°, preferably in the range 80° to 90°, and even more preferably, substantially equal to 90°.

8. A device according to any one of claims 1 to 6, wherein said fluidic junction (30) is a microfluidic flow focusing junction.

9. A device according to any preceding claim, wherein said first and second ducts present sections lying in the range 110% to 130% of the diameter $\underline{d}$ of the article to be encapsulated; said discharge duct(s) present sections of the order of 30% to 60% of the section of the first duct; and the distance(s) of the mouth(s) of said discharge duct(s) or pair(s) of discharge ducts from said junction lie(s) in the range 10% to 50% of the diameter of the article to be encapsulated.

10. A device according to any preceding claim, including an array of micro-pillars (450, 450') at said or each mouth of said discharge duct(s).

11. A device according to any preceding claim, including an array of micro-pillars (451) at the fluidic junction between the first and second ducts.

12. A device according to any preceding claim, presenting a planar structure, wherein said ducts are constituted by furrows etched or molded in a substrate.

13. A device according to any one of claims 1 to 11, presenting a three-dimensional structure, being constituted by a stack of molded or etched planar substrates ($S_1$ - $S_5$), with the first duct passing substantially perpendicularly through said substrate, while the second duct and the discharge duct(s) are formed at interfaces between substrates and parallel thereto.

14. A device according to any preceding claim, wherein the dimensions of said ducts are adapted to enable cells or bacteria to be encapsulated.

15. A microfluidic system for controlled encapsulation of particles of sub-millimetric dimensions or clusters of such particles, the system comprising:

   • a device (D) according to any preceding claim;
   • injector means ($P_1$) for injecting into the first duct of said device a first liquid phase containing in suspension particles of sub-millimetric dimensions or clusters of such particles for encapsulating, said liquid phase also containing at least one encapsulation agent;
   • discharge means for discharging the first liquid phase flowing in said first duct via said discharge duct(s); and
   • injector means ($P_2$) for injecting into the second duct of said device a second liquid phase that is immiscible with said liquid phase;

wherein said injector means for injecting the first and second liquid phases, and said discharge means for discharging the first liquid phase are adapted to enable a stable interface to be formed between the two immiscible liquid phases at said junction, and to enable said interface to rupture in the event of one or more of said discharge ducts being obstructed.

16. A microfluidic system according to claim 15, also including a gelling unit (E) connected to said second duct downstream from said junction, said unit being adapted to:

   • receive as input said second liquid phase containing in suspension the particles or particle clusters injected into said second duct and surrounded in film constituted by the first liquid phase; and
   • cause the encapsulation agent contained in said film to gel so as to constitute capsules containing said particles or particle clusters.

17. A microfluidic method for controlled encapsulation of sub-millimetric particles or clusters of such particles, the method comprising the steps consisting in:

   • injecting into the first duct of a device according to any one of claims 1 to 14 a first liquid phase containing in suspension particles or particle clusters for encapsulating, said liquid phase also containing at least one encapsulation agent;
   • discharging the liquid phase flowing in said first duct through said discharge duct(s); and
   • injecting into the second duct of said device a second liquid phase that is immiscible with said first liquid phase;

   the pressure that which the first and second liquid phases are injected and the discharge flow rate of the first liquid phase being selected in such a manner as to enable a stable interface to be formed between the two immiscible liquid phases at said junction, and to enable said interface to be ruptured and a controlled number of particles or particle clusters to be injected into said second duct in the event of one or more of said discharge ducts being obstructed.

18. A method according to claim 17, further including a step of gelling the encapsulation agent contained in a film constituted by said first liquid phase surrounding the particles or particle clusters injected into said second duct in order to form solid-walled capsules (K') enclosing said particles or particle clusters.

19. A method according to claim 17 or claim 18, wherein said particles are selected from cells, in particular human cells, and bacteria.

**Patentansprüche**

1. Mikrofluidvorrichtung (D) zur kontrollierten Einkapselung von Partikeln mit Submillimetergröße oder von Ansammlungen solcher Partikel, umfassend:

   - eine erste Mikrofluidleitung (10) zur Zuführung einer ersten flüssigen Phase ($\phi_1$), die Partikel oder Ansammlungen von Partikeln (C), die einzukapseln sind, in Suspension enthält;
   - eine zweite Mikrofluidleitung (20) zur Zirkulation einer zweiten flüssigen Phase ($\phi_2$), die mit der ersten flüssigen Phase nicht mischbar ist; wobei die erste Leitung in die zweite Leitung einmündet und eine Fluidverbindung (30, 30') mit dieser letztgenannten bildet;
   - wenigstens eine Mikrofluidleitung zum Abfließen (40, 40', 50, 50') der ersten flüssigen Phase, die in der ersten Leitung zirkuliert, die mit einer Einmündung (45, 45') versehen ist, welche, stromaufwärts der Verbindung angeordnet ist und empfindlich ist, wenigstens teilweise, durch ein Partikel oder Ansammlung von Partikeln in Suspension verstopft zu werden, wobei so ein Überdruck in der ersten Leitung hervorgerufen wird;

   wodurch Partikel oder Ansammlungen von Partikeln, die von der genannten Suspension entlang der ersten Leitung mitgeführt werden, in der Lage sind, auf der Höhe der Verbindung zu akkumulieren, auf der Höhe einer Grenzfläche (I) zwischen den zwei nicht mischbaren flüssigen Phasen abgefangen zu werden, bis sie durch den Überdruck, der durch die Verstopfung der Einmündung oder der Einmündungen der Leitung(en) zum Abfließen hervorgerufen wird, in die zweite Leitung eingespritzt werden.

2. Vorrichtung gemäß Anspruch 1, die wenigstens ein Paar Mikrofluidleitungen zum Abfließen umfasst, die an beiden Seiten der ersten Leitung angeordnet sind.

3. Vorrichtung gemäß einem der vorangehenden Ansprüche, die eine einzelne Leitung oder ein Paar Leitungen zum Abfließen umfasst.

4. Vorrichtung gemäß Anspruch 3, in der der Querschnitt der Einmündung der Leitung oder der Leitungen zum Abfließen und ihr Abstand von der Verbindung sowie der Querschnitt der ersten Leitung so gewählt sind, dass die Ankunft eines einzelnen Partikels oder einer Ansammlung von Partikeln auf der Höhe der Verbindung in der ersten Leitung einen genügenden Überdruck induziert, um die Einspritzung des Partikels oder der Ansammlung von Partikeln in die zweite Leitung zu erlauben.

**5.** Vorrichtung gemäß Anspruch 3, in der der Querschnitt der Einmündung der Leitung oder der Leitungen zum Abfließen und ihr Abstand von der Verbindung sowie der Querschnitt der ersten Leitung so gewählt sind, dass ein ausreichender Überdruck, um die Einspritzung eines Partikels oder einer Ansammlung von Partikeln in die zweite Leitung zu ermöglichen, nur erreicht wird, wenn eine vorbestimmte Anzahl von Partikeln oder Ansammlungen von Partikeln, die über eins und vorzugsweise zwischen 2 und 10 liegt, auf der Höhe der Verbindung durch die genannte Grenzfläche zwischen den zwei flüssigen nicht mischbaren Phasen abgefangen wird.

**6.** Vorrichtung gemäß einem der Ansprüche 1 oder 2, die wenigstens zwei Leitungen oder Paare von Leitungen zum Abfließen umfasst, deren Einmündungen in verschiedenen Abständen von der Verbindung gelegen sind, in der der Querschnitt der Einmündung der Leitung oder der Leitungen zum Abfließen und sein Abstand von der Verbindung sowie der Querschnitt der ersten Leitung so gewählt sind, dass ein ausreichender Überdruck, um eine Einspritzung eines Partikels oder einer Ansammlung von Partikeln in die zweite Leitung zu ermöglichen, nur erreicht wird, wenn die zwei Leitungen oder die Paare von Leitungen verstopft sind, wodurch die gleichzeitige Einspritzung einer Vielzahl von Partikeln oder Ansammlungen von Partikeln erreicht werden kann.

**7.** Vorrichtung gemäß einem der vorangehenden Ansprüche, in der die Fluidverbindung (30) eine Verbindung in "T"-Form ist, die erste und die zweite Leitung miteinander einen Winkel bilden, der zwischen 60° und 90°, vorzugsweise zwischen 80° und 90° und noch bevorzugter etwa 90° ist.

**8.** Vorrichtung gemäß einem der Ansprüche 1 bis 6, in der die Fluidverbindung (30') eine Verbindung zur Fokussierung zu einem Mikrofluidfluss ist.

**9.** Vorrichtung gemäß einem der vorangehenden Ansprüche, in der die ersten und zweiten Leitungen Querschnitte aufweisen, die zwischen 110 und 130% des Durchmessers d des einzukapselnden Objektes liegen; die Leitung oder die Leitungen zum Abfließen Querschnitte in der Größenordnung von 30 bis 60% des Querschnitts der ersten Leitung aufweisen und die Einmündungen der Leitung oder der Leitungen oder der Paare von Leitungen zum Abfließen der Verbindung zwischen 10 und 50% des Durchmessers des einzukapselnden Objekts liegen.

**10.** Vorrichtung gemäß einem der vorangehenden Ansprüche, die ein Netzwerk aus Mikrostützen (450, 450') auf der Höhe der Einmündung oder jeder Einmündung der Leitung oder der Leitungen zum Abfließen umfasst.

**11.** Vorrichtung gemäß einem der vorangehenden Ansprüche, die ein Netzwerk aus Mikrostützen (451) auf der Höhe der Fluidverbindung zwischen der ersten und der zweiten Leitung umfasst.

**12.** Vorrichtung gemäß einem der vorangehenden Ansprüche, die eine planare Struktur aufweist, in der die Leitungen durch Rillen gebildet werden, die in ein Substrat geritzt oder geformt sind.

**13.** Vorrichtung gemäß einem der Ansprüche 1 bis 11, die eine dreidimensionale Struktur aufweist und die durch einen Stapel planarer Substrate ($S_1$-$S_5$), die geformt oder geritzt sind, gebildet wird, in der die erste Leitung in etwa senkrechter Art die Substrate durchquert, während die zweite Leitung und die Leitung oder Leitungen zum Abfließen auf der Höhe der Grenzflächen zwischen Substraten und parallel zu diesen letztgenannten gebildet sind.

**14.** Vorrichtung gemäß einem der vorangehenden Ansprüche, in der die Abmessungen der Leitungen angepasst sind, um eine Einkapselung von Zellen oder Bakterien zu erlauben.

**15.** Mikrofluidsystem zur kontrollierten Einkapselung von Partikeln mit Submillimetergröße oder von Ansammlungen solcher Partikel, umfassend:

- eine Vorrichtung (D) gemäß einem der vorangehenden Ansprüche;
- Mittel ($P_1$) zur Einspritzung, in der ersten Leitung der Vorrichtung, einer ersten flüssigen Phase, die in Suspension Partikel mit Submillimetergröße oder Ansammlungen solcher Partikel, die einzukapseln sind, umfasst, wobei die flüssige Phase auch wenigstens ein Einkapselungsmittel enthält;
- Mittel zum Abfließen der ersten flüssigen Phase, die in der ersten Leitung zirkuliert, durch die Leitung oder die Leitungen zum Abfließen und
- Mittel zur Einspritzung ($P_2$), in die zweite Leitung der Vorrichtung, einer zweiten flüssigen Phase, die mit der ersten flüssigen Phase nicht mischbar ist;

in der die Mittel zur Einspritzung der ersten und der zweiten flüssigen Phase sowie die Mittel zum Abfließen der ersten flüssigen Phase angepasst sind, um die Bildung einer stabilen Grenzfläche zwischen den zwei flüssigen nicht mischbaren Phasen auf der Höhe der Verbindung sowie die Ruptur der Grenzfläche im Fall einer Verstopfung einer oder mehrerer der Leitungen zum Abfließen zu ermöglichen.

**16.** Mikrofluidsystem gemäß Anspruch 15, das auch eine Gelierungseinheit (E) umfasst, die mit der zweiten Leitung stromabwärts der Verbindung verbunden ist, wobei die Einheit angepasst ist, um:

- die zweite flüssige Phase, die Partikel oder Ansammlungen von Partikeln enthält, die in die zweite Leitung injiziert wurden, die von einem durch die erste flüssige Phase gebildeten Überzug umgeben sind, am Einlass aufzunehmen und
- die Gelierung des Einkapselungsmittels, das in dem Überzug enthalten ist, derart hervorzurufen, dass Kapseln gebildet werden, die die Partikel oder die Ansammlung von Partikeln enthalten, zu bilden.

17. Mikrofluidverfahren zur kontrollierten Einkapselung von Partikeln mit Submillimetergröße oder von Ansammlungen solcher Partikel, umfassend die Stufen, bestehend aus:

- Einspritzen, in die erste Leitung einer Vorrichtung gemäß einem der Ansprüche 1 bis 14, eine erste flüssige Phase, die Partikel oder Ansammlungen von Partikeln, die einzukapseln sind, in Suspension enthält, wobei die flüssige Phase auch wenigstens ein Einkapselungsmittel enthält;
- Abfließenlassen der flüssigen Phase, die in der ersten Leitung zirkuliert, durch die Leitung oder die Leitungen zum Abfließen und
- Einspritzen, in die zweite Leitung der Vorrichtung, eine zweite flüssige Phase, die mit der ersten flüssigen Phase nicht mischbar ist;

wobei die Drücke zur Einspritzung der ersten und der zweiten flüssigen Phase sowie der Beginn des Abfließens der ersten flüssigen Phase so gewählt werden, dass die Bildung einer stabilen Grenzfläche zwischen den zwei nicht mischbaren flüssigen Phasen auf der Höhe der Verbindung sowie die Ruptur der Grenzfläche und die Einspritzung in die zweite Leitung einer kontrollierten Zahl von Partikeln oder Ansammlungen von Partikeln im Fall der Verstopfung von einer oder mehreren der Leitungen zum Abfließen ermöglicht wird.

18. Verfahren gemäß Anspruch 17, das auch eine Stufe der Gelierung des Einkapselungsmittels, das in einem Überzug enthalten ist, der aus der ersten flüssigen Phase besteht, der die Partikel oder die Ansammlung von Partikeln, die in die zweite Leitung eingespritzt werden, umgibt, um Kapseln (K') mit fester Wand, die die Partikel oder die Ansammlung von Partikeln einschließt, zu bilden.

19. Verfahren gemäß einem der Ansprüche 17 oder 18, in dem die Partikel aus Zellen, insbesondere menschlichen, und Bakterien ausgewählt werden.

FIG.1

FIG.2

FIG.3

EP 2 119 775 B1

FIG.4a

FIG.4b

FIG.4c

FIG.4d

FIG.4e

16

FIG.5

FIG.6

FIG.7

FIG.8

**EP 2 119 775 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060051329 A
- WO 2005103106 A
- WO 2004037374 A
- WO 2006102258 A
- WO 200202316 A
- FR 0802575

**Littérature non-brevet citée dans la description**

- **ZIMMERMANN.** Fabrication of homogeneously cross-linked, functional alginate microcapsules validated by NMR-, CLSM-, and AFM-imaging. *Biomaterials,* 2003, vol. 24, 2083-2096
- **GOOSEN.** Electrostatic droplet generation for encapsulation of somatic tissue: assessment of high-voltage power supply. *Biotechnol. Prog.,* 1997, vol. 13, 497-502
- **SEIFERT.** Production of small, monodispersed alginate beads for cell immobilization. *Biotechnol.Prog.,* 1997, vol. 13, 562-568
- **WORKMAN et al.** Microfluidic chip-based synthesis of alginate microspheres for encapsulation of immortalized human cells. *Biomicrofluidics,* 2007, 1
- **SUGIURA et al.** Size control of calcium alginate beads containing living cells using micronozzle array. *Biomaterials,* 2005, vol. 26, 3327-3331
- **T. THORSEN et al.** Dynamic pattern formation in a vesicle-generating microfluidic device. *Physical Review Letters,* 2001, vol. 86 (18), 4163-4166
- **S.L. ANNA ; N. BONTOUX ; H.A. STONE.** Formation of dispersions using "flow focusing" in microchannels. *Applied Physics Letters,* 2003, vol. 82 (3), 364-366